# EUROPEAN PATENT SPECIFICATION

(11) **EP 2 513 046 B1**
(45) Date of publication and mention of the grant of the patent: **08.07.2015**
(21) Application number: 10809329.5
(22) Date of filing: 13.12.2010
(51) Int. Cl.: C07C 253/30, C07D 257/04

(54) **PROCESS FOR THE PREPARATION OF 4-BROMOMETHYL-[1,1'-BIPHENYL]-2'-CARBONITRILE**
VERFAHREN ZUR HERSTELLUNG VON 4-BROMMETHYL-[1,1'-BIPHENYL]-2'-CARBONITRIL
PROCÉDÉ POUR LA PRÉPARATION DU 4-BROMOMÉTHYL-[1,1'-BIPHÉNYLE]-2'-CARBONITRILE

(30) Priority: 16.12.2009 HU P0900788
(43) Date of publication of application: 24.10.2012
(73) Proprietor: SANOFI, 75008 Paris (FR)
(72) Inventor: HUSZÁR, Csaba, 1045 Budapest (HU); ÁRVAI, Géza, 1045 Budapest (HU); HEGEDUS, Adrienn, 1045 Budapest (HU)
(74) Representative: Ori, Janos
(86) International application number: PCT/HU2010/000141
(87) International publication number: WO 2011/073703

(56) References cited:
- GB-A- 1 563 164
- GB-A- 2 306 956
- US-A- 2 535 131
- US-A1- 2006 041 147
- US-B1- 6 214 999

## Description

The subject of the invention is novel method for the preparation of the compounds of the general formula (I) 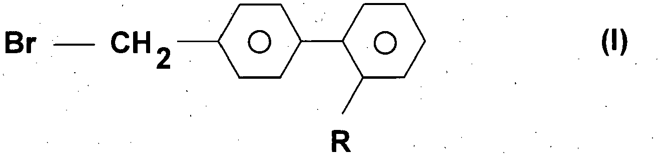
- where in the formula R represents cyano group, - COOR¹ or -CONR²R³ group - where R¹, R² and R³ stand for identical or non-identical substituents chosen from the following atom or groups: hydrogen atom, straight or branched C₁₋₇ alkyl group, C₃₋₆ cycloalky group; or in one given case a tetrazolyl group substituted with a C₁₋₄ alkyl group or triphenylmethyl group - by bromination of the compounds of the general formula (II) 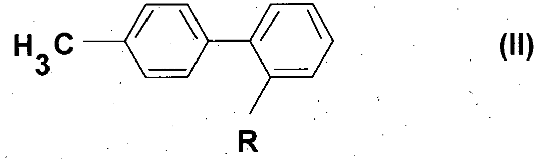
- where the meaning of R is as given above - by the method defined in the invention.

The compounds of the general formula (I) are useful intermediates to various biologically active compounds, as for example the blood pressure reducing and angiotensine antagonistic irbesartan, telmisartan and olmesartan (EP454511, EP-503214A, EP503785A).

The preparation of the compounds of the general formula (I) by bromination of the compounds of the general formula (II) and their isolation in pure form involve difficulties. The previous known methods give rise also to a byproduct, which is the appropriate 4,4-dibromo derivative of the compounds of the general formula (I). These 4,4-dibromo derivatives are difficult to separate from the compounds of the general formula (I). Furthermore, the bromination agents and the procedures applied in the previous known methods have several drawbacks.

We aimed to work out a bromination method which avoids the drawbacks of the known methods, allows the preparation of the compounds of the general formula (I) in a simple and environment-friendly way from the compounds of the general formula (II), and which is also suitable for scale-up.
- We have found that if the compounds of the general formula (II) - where in the formula R represents cyano group, - COOR¹ or -CONR²R³ group - where R¹, R² and R³ stand for identical or non-identical substituents chosen from the following atom or groups: hydrogen atom, straight or branched C₁₋₇ alkyl group, C₃₋₆ cycloalky group; or in one given case a tetrazolyl group substituted with a C₁₋₄ alkyl group or triphenylmethyl group -
are reacted with one of the following reagent pairs, as bromine sources: bromate and pyrosulfite or bromide and percarbonate or bromide and perborate, in aqueous-organic binary systems, then the resulting compounds of the general formula (I) contain only a very small amount of the 4,4-dibromo by-products from which the product can be well and easily separated. Furthermore, the applied brominating agents are inexpensive and easy to handle.

In the course of the above novel method, alkanes, cycloalkanes, aromatic hydrocarbons, halogenated alkanes, halogenated aromatic hydrocarbons or aliphatic carboxylic acid esters can be used as solvents. Such organic phases comprise: pentane, hexane, heptane, octane, cyclohexane, cycloheptane, methylcyclohexane, benzene, toluene, dichloromethane, dichloroethane, dibromomethane, bromochloromethane, carbon tetrachloride, ethyl acetate or methyl acetate.

The components of the bromine source can be chosen from sodium bromate, potassium bromate or other alkali metal bromates, from sodium hydrogen sulfite, potassium hydrogen sulfite or other alkali metal hydrogen sulfites, from sodium pyrosulfite, potassium pyrosulfite or other alkali metal pyrosulfite, in such a way that to a bromate salt either a hydrogen sulfite salt or a pyrosulfite salt is chosen.

The bromination according to the invention can be carried out by dissolving the alkali bromate salt in water and adding to this solution a compound of the general formula (II) dissolved in an organic solvent. To this mixture is then added the aqueous solution of the alkali metal hydrogen sulfite or pyrosulfite. From the reaction mixture the compound of the general formula (I) can be isolated by methods known *per se,* the variations of the methods are demonstrated by the examples. The alkali metal hydrogen sulfite or pyrosulfite salt solutions are added under the necessary cooling.

To prepare the mixture of hydrogen bromide and or any other bromide with the percarbonate or perborate, any stable percarbonate or perborate can be used. The use of alkali metal percarbonates or perborates, like sodium percarbonate or sodium perborate is one embodiment of the present invention, but other metal carbonates or perborates can also be applied.

The reaction is performed at a temperature between 5 °C and 80 °C.

The components of the bromine source are applied in equimolar or non-equimolar ratio.

Further details of the invention are demonstrated by the examples, without limiting the claims to the examples.

### EXAMPLES

### Example 1.

To the solution of 1.35 g (9 mmol) NaBrO₃ (sodium bromate) in 4.5 ml water, 0.58 g (3 mmol) 4-methyl-[1,1'-biphenyl]-2-carbonitrile dissolved in 6 ml ethyl acetate and then, in 15 minutes, under cooling 0.93 g (9 mmol) NaHSO₃ (sodium hydrogen sulfite) dissolved in 9 ml water were added. The reaction mixture was stirred at room temperature for 4 hours. The resulting suspension was filtered, to obtain 0.76 g (93 %) 4-bromomethyl-[1,1-biphenyl]-2-carbonitrile.

### Example 2.

To the solution of 1.35 g (9 mmol) NaBrO₃ in 4.5 ml water, 0.58 g (3 mmol) 4-methyl-[1,1'-biphenyl]-2-carbonitrile dissolved in 20 ml cyclohexane and then in 15 minutes under cooling 0.93 g (9 mmol) NaHSO₃ in 9 ml water were added. Further on the procedure as described in Example 1 was followed, to obtain 0.76 g (93 %) 4-bromomethyl-[1,1'- biphenyl]-2-carbonitrile.

### Example 3.

The solution of 1.35 g (9 mmol) NaBrO₃ in 4.5 ml water was added to 0.58 g (3 mmol) 4-methyl-[1,1-biphenyl]-2-carbonitrile dissolved in 6 ml carbon tetrachloride and to this mixture was added in 15 minutes under cooling 0.93 g (9 mmol) NaHSO₃ in 9 ml water. Further on the procedure as described in Example 1. was followed, to obtain 0.76 g (93 %) 4-bromomethyl-[1,1'-biphenyl]-2'-carbonitrile.

### Example 4.

The solution of 1.35 g (9 mmol) NaBrO₃ in 4.5 ml water was added to 0.58 g (3 mmol) 4-methyl-[ 1,1'-biphenyl]-2 -carbonitrile dissolved in 6 ml dibromomethane and to this mixture were added in 15 minutes under cooling 0.93 g (9 mmol) NaHSO₃ in 9 ml water. Further on the procedure as described in Example 1. was followed, to obtain 0.76 g (93 %) 4-bromomethyl-[1,1'-biphenyl]-2-carbonitrile.

### Example 5.

The solution of 1.35 g (9 mmol) NaBrO₃ in 4.5 ml water was added to 0.58 g (3 mmol) 4-methyl-[1,1'-biphenyl]-2-carbonitrile dissolved in 6 ml dichloromethane and to this mixture were added in 15 minutes, under cooling 0.93 g (9 mmol) NaHSO₃ in 9 ml water. Further on the procedure as described in Example 1. was followed, to obtain 0.76 g (93 %) 4-bromomethyl-[1,1-biphenyl]-2-carbonitrile.

### Example 6.

The solution of 1.35 g (9 mmol) NaBrO₃ in 4.5 ml water was added to 0.58 g (3 mmol) 4-methyl-[1,1'-biphenyl]-2 -carbonitrile in 6 ml monochlorobenzene and to this mixture were added in 15 minutes under cooling 0.93 g (9 mmol) NaHSO₃ in 9 ml water. Further on the procedure as described in Example 1. was followed, to obtain 0.76 g (93%) 4-bromomethyl-[1,1'-biphenyl]-2-carbonitrile.

### Example 7.

To the solution of 1.35 g (9 mmol) NaBrO₃ in 4.5 ml water was added 1.44 g (3 mmol) *N*-triphenylmethyl-5-(4-methyl-[1,1'-biphenyl]-2'-yl)-1*H*-tetrazole dissolved in 6 ml dichloroethane. To this mixture was then added under cooling the solution of 0.93 g (9 mmol) NaHSO₃ in 9 ml water. Further on the procedure as described in Example 1. was followed, to obtain 1.50 g (90 %) 5-{(4-bromomethyl)-[1,1-biphenyl]-2-yl}-*N*-triphenylmethyl-1*H*-tetrazole.

### Example 8.

To 317 g (2.05 mol) sodium bromate dissolved in 100 ml water the solution of 193 g (1 mol) 4-methyl-[1,1'-biphenyl]-2'-carbonitrile in 250 ml ethyl acetate was added. To this mixture was then added dropwise, in 1 hour, at 15-20 °C, under cooling the solution of 494 g sodium pyrosulfte (Na₂S₂O₅) in 500 ml water. The resulting suspension was stirred at 20-25 °C for 4 hours. The solid material was filtered off, washed with cold water and dried. 253 g (93 %) 4-bromomethyl-[1,1 -biphenyl]-2-carbonitrile was obtained in the form of white crystals.

### Example 9.

To the solution of 1.35 g (9 mmol) NaBrO₃ in 4.5 ml water first 0.58 g (3 mmol) 4-methyl-[1,1'-biphenyl]-2'-carbonitrile dissolved in 6 ml methylcyclohexane, then in 15 minutes, under cooling, the solution of 0.93 (9 mmol) NaHSO₃ in 9 ml water were added. Further on the procedure as described in Example 1. was followed, to obtain 0.71 g (92 %) 4-bromomethyl-[1,1-biphenyl]-2-carbonitrile.

### Example 10.

To the solution of 33.8 g (0.224 mol) sodium bromate in 112.5 ml water 14.5 g (0.075 mol) 4-methyl-[1,1-biphenyl]-2-carbonitrile dissolved in 75 ml ethyl acetate was added at 20 °C. The reaction mixture was cooled to 0°C and at that temperature the solution of 23.25 g (0.122 mol) sodium pyrosulfite (Na₂S₂O₅) in 225 ml water was added. The mixture was heated to 15°C and stirred at that temperature for 5 hours. The 4-bromomethyl-[1,1-biphenyl]-2'-carbonitrile product precipitates from the reaction mixture, it is filtered off, washed with ethyl acetate and dried at 30°C in vacuum to obtain 18.72 g (91.7 %) product in a purity of 98.9 % by HPLC.

The 4,4-dibromomethyl-[1,1'-biphenyl]-2'-carbonitrile content of the obtained product is less than 0.2%.

### Example 11.

To the solution of 16.2 g (0.107 mol) sodium bromate in 54 ml water 7.0 g (0.036 mol) 4-methyl-[1,1'-biphenyl]-2'-carbonitrile dissolved in 70 ml methylcyclohexane was added at 20 °C. The reaction mixture was cooled to 0°C and 11.2 g (0.058 mol) Na₂S₂O₅ dissolved in 108 ml water was added to it at 0°C. The reaction mixture was heated to 15 °C and stirred at that temperature for 5 hours. The 4-bromomethyl-[1,1'-biphenyl]-2'-carbonitrile, product precipitated from the reaction mixture, it was filtered off, washed with methyl acetate and dried at 30°C in vacuum, to obtain 8.76 g (89.4 %) product in a purity of 98.4% (HPLC).

The 4,4-dibromomethyl-[1,1'-biphenyl]-2'-carbonitrile content of the product is less than 0.2 %.

### Example 12.

To the mixture of 500 g 4-methyl-[1,1'-biphenyl]-2'-carbonitrile, 1000 ml dichloromethane, 1000 ml water and 210 g sodium percarbonate was added dropwise, at 25 °C in 1 hour 390 ml of 9 N concentration aqueous hydrogen bromide solution. The mixture was stirred at 40 °C for 6 hours and cooled to room temperature. The organic phase was separated, washed twice with 300 ml of water, then the solution was concentrated until a solid material started to precipitate. The mixture was cooled to 5 °C and the resulting suspension was stirred at that temperature for 1 hour. The solid material was filtered off, washed with hexane and dried, to obtain 652 g (92 %) 4-bromomethyl-[1,1'-biphenyl]-2'-carbonitrile product in the form of white crystals.

### Example 13.

The procedure as described in Example 12. was followed, but the reaction was performed at 70 °C, dichloroethane was used as solvent and stirring was continued for 10 hours. The reaction mixture was worked up as described in Example 12, 550 g (78 %) 4-bromomethyl-[1,1'-biphenyl]-2'-carbonitrile was obtained in the form of white crystals.

### Example 14.

The procedure as described in Example 12. was followed, but the reaction was carried out in 1500 ml hexane at 40 °C and stirring was continued for 7 hours. The reaction mixture was worked up as described in Example 12. 634.6 g (90 %) 4-bromomethyl-[1,1'-biphenyl]-2'-carbonitrile was obtained in the form of white crystals.

### Example 15.

The procedure as described in Example 12. was followed, but the reaction was performed at 25 °C in 1,1,1-trichloroethane as solvent and stirring was continued for 10 hours. The reaction mixture was worked up as described in Example 12. 600 g (86 %) 4-bromomethyl-[1,1'-biphenyl]-2'-carbonitrile was obtained in the form of white crystals.

### Example 16.

10 g *N*-*tertiary*-butyl-5-(4-methyl-[1,1'-biphenyl]-2'-yl)-1*H*-tetrazole, 100 ml dichloromethane, 100 ml water, 4.5 g sodium percarbonate and 5.5 ml 9 N concentration aqueous hydrogen bromide solution were mixed and stirred at 35 °C for 6 hours. The mixture was then cooled to room temperature, the phases were separated and the aqueous layer was washed with 2x20 ml water. The solution was evaporated to almost dryness, to the residue 50 ml hexane was added and the mixture was stirred for 30 minutes. The precipitated solid material was filtered off, washed with hexane and dried, to obtain 11.5 g (91 %) 5-(4-bromomethyl-[1,1'-biphenyl]-2'-yl)-*N-tertiary*-butyl-1*H*-tetrazole.

### Example 17.

Starting from 16.4 g *N*-triphenylmethyl-5-(4-methyl-[1,1'-biphenyl]-2'-yl)-1*H-*tetrazole the reaction was carried out according to Example 16, but stirring was performed at 25 °C for 10 hours. After a work-up procedure as described in Example 16., 17.1 g (90 %) 5-(4-bromomethyl-[1,1'-biphenyl]-2'-yl)-*N-*triphenylmethyl-1*H*-tetrazole was obtained in the form of white crystals.

### Example 18.

To the mixture of 500 g 4-methyl-[1,1'-biphenyl]-2'-carbonitrile, 200 ml ethyl acetate, 500 ml water and 210 g sodium percarbonate in 1 hour, at 25 °C 390 ml 9 N aqueous hydrogen bromide solution was added dropwise. The mixture was stirred at 40 °C for 6 hours and cooled to room temperature. The resulting suspension was filtered, the solid material was washed with cold water and covered with hexane. 659 g (93 %) 4-bromomethyl-[1,1'-biphenyl]-2'-carbonitrile was obtained.

## Claims

1. Process for the preparation of the compounds of the general formula (I) 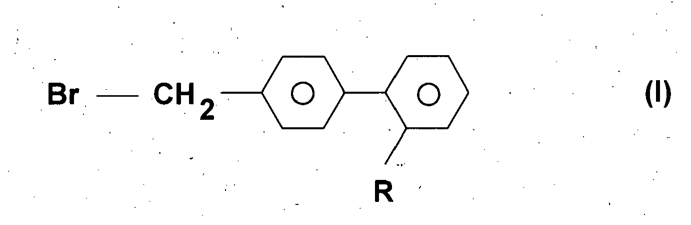
- where in the formula R represents cyano group, - COOR¹ or -CONR²R³ group - where R¹, R² and R³ stand for identical or non-identical substituents chosen from the following atom or groups: hydrogen atom, straight or branched C₁₋₇ alkyl group, C₃₋₆ cycloalkyl group; or in one given case a tetrazolyl group substituted with a C₁₋₄ alkyl group or triphenylmethyl group - c**h**aracterized **in that** a compound of the general formula (II) 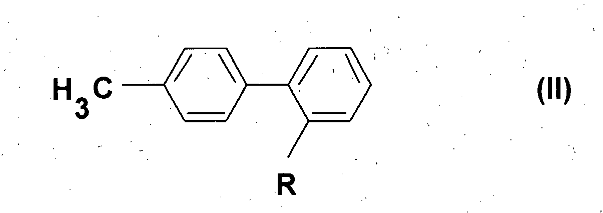
- where the meaning of R is as defined above - is reacted with one of the following reagent pairs as bromine sources: bromate with hydrogen sulfite, or bromate with pyrosulfite, or bromide with percarbonate, or bromide with perborate, in aqueous-organic binary systems, and optionally the resulting compound of the general formula (I) is isolated from the reaction mixture by a method known *per se.*

2. The process as defined in claim 1., **characterized in that** as for organic solvent, aliphatic carboxylic acid esters are used.

3. The process as defined in claim 1., **characterized in that** as for organic solvent, alkanes or cycloalkanes or aromatic hydrocarbons are used.

4. The process as defined in claim 1., **characterized in that** as for organic solvent, halogenated alkanes or halogenated aromatic hydrocarbons are used.

5. The process as defined in claim 2., **characterized in that** as for alifatic carboxylic acid ester, ethyl acetate or methyl acetate is used.

6. The process as defined in claim 3., **characterized in that** as for cycloalkane, cyclohexane or methylcyclohexane is used.

7. The process as defined in claim 4., **characterized in that** as for halogenated alkane, dichloromethane, dichloroethane, 1,1,1-trichloroethane, dibromoethane, bromochloromethane or carbon tetrachloride are used.

8. The process as defined in claim 4., **characterized in that** as for halogenated aromatic hydrocarbon, monochlorobenzene is used.

9. The process as defined in claim 1. **characterized in that** the bromination reaction is performed at a temperature between 5 C°-80 C°.

10. The process as defined in claim 1., **characterized in that** the starting material is a compound of the general formula (II) where R represents cyano- or 1-*tertiary*-butyl-tetrazol-5-yl- or 2-*tertiary*-butyl-tetrazol-5-yl- or 1-triphenylmethyl-tetrazol-5-yl- or 2-triphenylmethyl-tetrazol-5-yl group.

11. The process as defined in any of claims 1. - 10. **characterized in that** the reagent pair serving as the bromine source is a mixture of MeBrO₃/MeHSO₃ or MeBrO₃/Me₂S₂O₅ - where the meaning of Me is alkali metal.

12. The process as defined in any of claims 1. - 10. **characterized in that** the reagent pair serving as the bromine source is a mixture consisting of hydrogen bromide and a metal percarbonate.

13. The process as defined in claim 11. **characterized in that** the mixture of sodium bromate/sodium hydrogen sulfite or sodium bromate/sodium pyrosulfite is used.

14. The process as defined in claim 12. **characterized in that** hydrogen bromide/sodium percarbonate mixture is used.

## Patentansprüche

1. Verfahren zur Herstellung der Verbindungen der allgemeinen Formel (I) 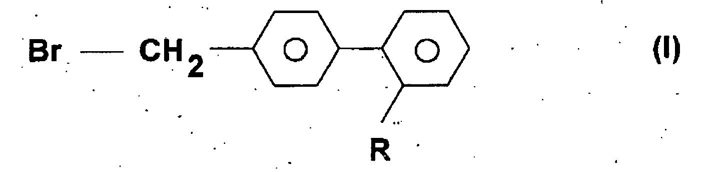
- wobei in der Formel R für eine Cyano-, -COOR¹- oder -CONR²R³-Gruppe, wobei R¹, R² und R³ für gleiche oder verschiedene Substituenten stehen, die aus dem folgenden Atom bzw. den folgenden Gruppen ausgewählt sind: Wasserstoffatom, gerade oder verzweigte C₁₋₇-Alkylgruppe, C₃₋₆-Cycloalkylgruppe; oder in einem gegebenen Fall eine Tetrazolylgruppe, die durch eine C₁₋₄-Alkylgruppe oder Triphenylmethylgruppe substituiert ist, steht, **dadurch gekennzeichnet, dass** man eine Verbindung der allgemeinen Formel (II) 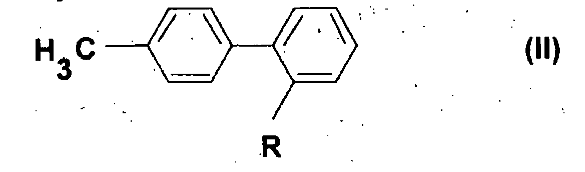
- wobei R die oben angegebene Bedeutung besitzt, in wässrig-organischen binären Systemen mit einem der folgenden Reagentienpaare als Bromquellen umsetzt: Bromat mit Hydrogensulfit oder Bromat mit Pyrosulfit oder Bromid mit Percarbonat oder Bromid mit Perborat, und gegebenenfalls die resultierende Verbindung der allgemeinen Formel (I) nach einer an sich bekannten Methode aus dem Reaktionsgemisch isoliert.

2. Verfahren nach Anspruch 1, **dadurch gekennzeichnet, dass** man als organisches Lösungsmittel aliphatische Carbonsäureester verwendet.

3. Verfahren nach Anspruch 1, **dadurch gekennzeichnet, dass** man als organisches Lösungsmittel Alkane oder Cycloalkane oder aromatische Kohlenwasserstoffe verwendet.

4. Verfahren nach Anspruch 1, **dadurch gekennzeichnet, dass** man als organisches Lösungsmittel halogenierte Alkane oder halogenierte aromatische Kohlenwasserstoffe verwendet.

5. Verfahren nach Anspruch 2, **dadurch gekennzeichnet, dass** man als aliphatischen Carbonsäureester Essigsäureethylester oder Essigsäuremethylester verwendet.

6. Verfahren nach Anspruch 3, **dadurch gekennzeichnet, dass** man als Cycloalkan Cyclohexan oder Methylcyclohexan verwendet.

7. Verfahren nach Anspruch 4, **dadurch gekennzeichnet, dass** man als halogeniertes Alkan Dichlormethan, Dichlorethan, 1,1,1-Trichlorethan, Dibromethan, Bromchlormethan oder Tetrachlorkohlenstoff verwendet.

8. Verfahren nach Anspruch 4, **dadurch gekennzeichnet, dass** man als halogenierten aromatischen Kohlenwasserstoff Monochlorbenzol verwendet.

9. Verfahren nach Anspruch 1, **dadurch gekennzeichnet, dass** man die Bromierungsreaktion bei einer Temperatur zwischen 5°C-80°C durchführt.

10. Verfahren nach Anspruch 1, **dadurch gekennzeichnet, dass** es sich bei dem Ausgangsstoff um eine Verbindung der allgemeinen Formel (II), wobei R für eine Cyano- oder 1-tert-Butyl-tetrazol-5-yl- oder 2-tert-Butyl-tetrazol-5-yl- oder 1-Triphenylmethyl-tetrazol-5-yl- oder 2-Triphenylmethyl-tetrazol-5-yl-gruppe steht, handelt.

11. Verfahren nach einem der Ansprüche 1-10, **dadurch gekennzeichnet, dass** es sich bei dem als Bromquelle dienenden Reagentienpaar um eine Mischung von MeBrO₃/MeHSO₃ oder MeBrO₃/Me₂S₂O₅ handelt, wobei Me ein Alkalimetall bedeutet.

12. Verfahren nach einem der Ansprüche 1-10, **dadurch gekennzeichnet, dass** es sich bei dem als Bromquelle dienenden Reagentienpaar um eine Mischung von Bromwasserstoff und einem Metallpercarbonat handelt.

13. Verfahren nach Anspruch 11, **dadurch gekennzeichnet, dass** man eine Mischung von Natriumbromat/Natriumhydrogensulfit oder Natriumbromat/Natriumpyrosulfit verwendet.

14. Verfahren nach Anspruch 12, **dadurch gekennzeichnet, dass** man eine Mischung von Bromwasserstoff und Natriumpercarbonat verwendet.

## Revendications

1. Procédé pour la préparation des composés représentés par la formule générale (I) 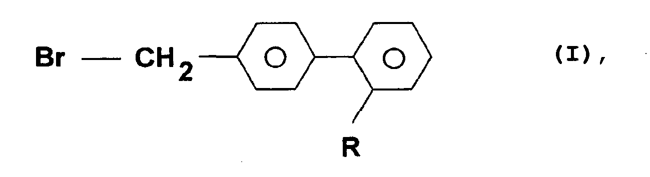 dans la formule R représentant un groupe cyano, -COOR¹ ou -CONR²R³, R¹, R² et R³ représentant des substituants identiques ou non identiques choisis parmi l'atome ou les groupes suivants : l'atome d'hydrogène, un groupe alkyle en C₁₋₇ droit ou ramifié, un groupe cycloalkyle en C₃₋₆ ; ou dans un cas donné un groupe tétrazolyle substitué par un groupe alkyle en C₁₋₄ ou un groupe triphénylméthyle,
**caractérisé en ce qu**'un composé représenté par la formule générale (II) 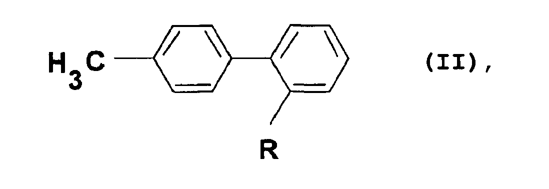 la signification de R étant telle que définie ci-dessus, est amené à réagir avec l'une des paires de réactifs suivantes en tant que sources de brome : du bromate avec de l'hydrogénosulfite ou du bromate avec du pyrosulfite ou du bromure avec du percarbonate ou du bromure avec du perborate, dans des systèmes binaires aqueux-organiques, et facultativement le composé représenté par la formule générale (I) résultant est isolé du mélange réactionnel par une technique connue en soi.

2. Procédé tel que défini dans la revendication 1, **caractérisé en ce que** comme solvant organique, des esters d'acides carboxyliques aliphatiques sont utilisés.

3. Procédé tel que défini dans la revendication 1, **caractérisé en ce que** comme solvant organique, des alcanes ou cycloalcanes ou des hydrocarbures aromatiques sont utilisés.

4. Procédé tel que défini dans la revendication 1, **caractérisé en ce que** comme solvant organique, des alcanes halogénés ou des hydrocarbures aromatiques halogénés sont utilisés.

5. Procédé tel que défini dans la revendication 2, **caractérisé en ce que** comme ester d'acide carboxylique aliphatique, de l'acétate d'éthyle ou de l'acétate de méthyle est utilisé.

6. Procédé tel que défini dans la revendication 3, **caractérisé en ce que** comme cycloalcane, du cyclohexane ou du méthylcyclohexane est utilisé.

7. Procédé tel que défini dans la revendication 4, **caractérisé en ce que** comme alcane halogéné, du dichlorométhane, du dichloroéthane, du 1,1,1-trichloroéthane, du dibromoéthane, du bromochlorométhane ou du tétrachlorure de carbone sont utilisés.

8. Procédé tel que défini dans la revendication 4, **caractérisé en ce que** comme hydrocarbure aromatique halogéné, du monochlorobenzène est utilisé.

9. Procédé tel que défini dans la revendication 1, **caractérisé en ce que** la réaction de bromation est effectuée à une température comprise entre 5 °C et 80 °C.

10. Procédé tel que défini dans la revendication 1, **caractérisé en ce que** la matière de départ est un composé représenté par la formule générale (II), R représentant un groupe cyano- ou 1-*tert*-butyltétrazol-5-yl- ou 2-*tert*-butyltétrazol-5-yl- ou 1-triphénylméthyltétrazol-5-yl- ou 2-triphénylméthyltétrazol-5-yl-.

11. Procédé tel que défini dans l'une quelconque des revendications 1-10 **caractérisé en ce que** la paire de réactifs servant de source de brome est un mélange de MeBrO₃/MeHSO₃ ou de MeBrO₃/Me₂S₂O₅, Me désignant un métal alcalin.

12. Procédé tel que défini dans l'une quelconque des revendications 1-10 **caractérisé en ce que** la paire de réactifs servant de source de brome est un mélange constitué de bromure d'hydrogène et d'un percarbonate de métal.

13. Procédé tel que défini dans la revendication 11 **caractérisé en ce que** le mélange de bromate de sodium/hydrogénosulfite de sodium ou de bromate de sodium/pyrosulfite de sodium est utilisé.

14. Procédé tel que défini dans la revendication 12 **caractérisé en ce que** du mélange bromure d'hydrogène/percarbonate de sodium est utilisé.
